(19) Europäisches Patentamt
European Patent Office
Office européen des brevets

(11) Veröffentlichungsnummer: **0 167 763**
**B1**

# (12) EUROPÄISCHE PATENTSCHRIFT

(45) Veröffentlichungstag der Patentschrift: **03.08.88**

(21) Anmeldenummer: **85106250.5**

(22) Anmeldetag: **22.05.85**

(51) Int. Cl.⁴: **C 07 D 215/56**, C 07 D 401/04, C 07 D 413/04, A 61 K 31/47, A 61 K 31/495, A 61 K 31/535, C 07 D 417/04, A 61 K 31/54, C 07 D 409/14

(54) **7-Amino-1-cyclopropyl-6,8-dihalogen-1,4-dihydro-4-oxo-3-chinolincarbonsäuren, Verfahren zur ihrer Herstellung sowie diese enthaltende antibakterielle Mittel.**

(30) Priorität: **04.06.84 DE 3420743**

(43) Veröffentlichungstag der Anmeldung: **15.01.86 Patentblatt 86/3**

(45) Bekanntmachung des Hinweises auf die Patenterteilung: **03.08.88 Patentblatt 88/31**

(84) Benannte Vertragsstaaten: **AT BE CH DE FR GB IT LI LU NL SE**

(56) Entgegenhaltungen:
**EP - A - 0 049 355**
**EP - A - 0 126 355**
**BE - A - 899 399**
**GB - A - 2 057 440**
**US - A - 4 398 029**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeeker Strasse 46, D-5620 Velbert 15 (DE)**
Erfinder: **Metzger, Karl Georg, Dr., Pahlkestrasse 75, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 7-Amino-1-cyclopropyl-6,8-dihalogen-1,4-dihydro-4-oxo-3-chinolincarbonsäuren, Verfahren zu ihrer Herstellung sowie diese enthaltende antibakterielle Mittel.

Antibakteriell wirksame Chinoloncarbonsäuren sind aus EP-A-049 355, US-A-4 398 029 und GB-A-2 057 440 bekannt. Obwohl insbesondere in der EP-A-049 355 hochwirksame Chinoloncarbonsäuren beschrieben werden, sind gegenüber bestimmten Keimen Wirkungssteigerungen erwünscht.

Gefunden wurden die neuen 7-Amino-1-cyclopropyl-6,8-dihalogen-1,4-dihydro-4-oxo-3-chinolincarbonsäuren der Formel (I),

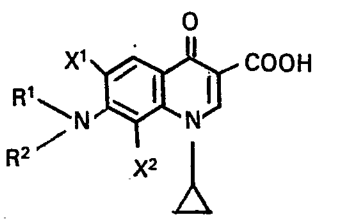

(I)

in welcher

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatom, an das sie gebunden sind, einen 5- oder 6-gliedrigen heterocyclischen Ring bilden, der als Ringglied zusätzlich die Atome oder Gruppen –O–, –S–, –SO–, $-SO_2-$, $>N-R^3$ oder $-CO-N-R^3$ enthalten kann und der gegebenenfalls an den Kohlenstoffatomen ein- bis dreifach durch $C_1-C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino oder Ethylamino substituiert sein kann, wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine oder zwei Hydroxy-, Alkoxy-, Alkylamino- oder Dialkylaminogruppen mit 1 bis 3 Kohlenstoffatomen für einen Alkylrest, die Cyanogruppe, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^5$ geradkettiges oder verzweigtes Alkyl mit 1 bis 3 Kohlenstoffatomen darstellt, und deren pharmazeutisch verwendbaren Hydrate, Säureadditionssalze, Alkali-, Erdalkali- und Guanidiniumsalze, die eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatom, an das sie gebunden sind, einen 5- oder 6-gliedrigen heterocyclischen Ring bilden können, der als Ringglied zusätzlich die Atome oder Gruppen –S–, $-SO_2-$, $N-R^3$ oder $-CO-N-R^3$ enthalten kann und der gegebenenfalls an den Kohlenstoffatomen ein- bis zweifach durch $C_1-C_3$-Alkyl, Cyclohexyl, gegebenenfalls durch Chlor, Fluor, Brom, Methyl, Phenyl, Hydroxy, Methoxy, Benzyloxy, Nitro oder Piperidino ein oder zweifach substituiertes Phenyl, 2-Thienyl, Hydroxy, Amino oder Methylamino substituiert sein kann, wobei

$R^3$ für Wasserstoff, eine verzweigte oder unverzweigte Alkyl-, Alkenyl- oder Alkinylgruppe mit 1 bis 4 Kohlenstoffatomen, die gegebenenfalls durch eine oder zwei Hydroxygruppen substituiert sein kann, einen Phenacylrest, einen Oxoalkylrest mit bis zu 5 Kohlenstoffatomen sowie für einen Rest $COR^4$, steht, wobei

$R^4$ Wasserstoff, geradkettiges oder verzweigtes Alkyl mit 1 bis 3 Kohlenstoffatomen, Alkoxy mit 1 bis 3 Kohlenstoffatomen, Amino, Alkylamino oder Dialkylamino mit 1 bis 3 Kohlenstoffatomen im Alkylteil bedeutet.

Besonders bevorzugt sind diejenigen Verbindungen der Formel (I), in denen

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatom, an das sie gebunden sind, einen 5- oder 6-gliedrigen heterocyclischen Ring bilden können, der als Ringglied zusätzlich ein Sauerstoffatom oder die Gruppen $N-R^3$ oder $-CO-N-R^3$ enthalten kann und der gegebenenfalls an den Kohlenstoffatomen ein- bis zweifach durch $C_1-C_2$-Alkyl, Cyclohexyl, gegebenenfalls durch Chlor, Fluor, Methyl, Phenyl, Hydroxy, Methoxy, Benzyloxy, Nitro oder

Piperidino substituiertes Phenyl, 2-Thienyl oder Hydroxy substituiert sein kann, wobei

$R^3$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxoalkylrest mit bis zu 4 Kohlenstoffatomen sowie einen Rest $COR^4$ steht, wobei

$R^4$ Wasserstoff oder Alkyl mit ein oder zwei Kohlenstoffatomen bedeutet.

Weiterhin wurde gefunden, dass man die Verbindungen der Formel (I) erhält, wenn man die 1-Cyclopropyl- 7-halogen- 1,4-dihydro- 4-oxo-3-chinolincarbonsäuren der Formel (II),

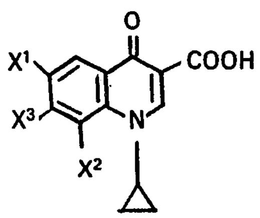

(II)

in welcher

$X^1$ und $X^2$ die oben angegebene Bedeutung haben, und

$X^3$ für Halogen, bevorzugt Chlor oder Fluor, steht, mit Aminen der Formel (III),

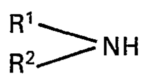

(III)

in welcher

$R^1$ und $R^2$ die oben angegebene Bedeutung haben, gegebenenfalls in Gegenwart von Säurebindern umsetzt (Methode A).

Erfindungsgemässe Verbindungen der Formel (I) können auch erhalten werden, indem man eine 7-(1-Piperazinyl)-3-chinoloncarbonsäure der Formel (IV),

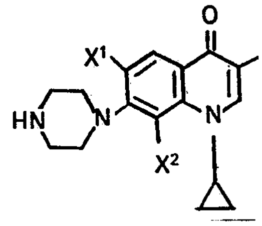

(IV)

in welcher

$X^1$ und $X^2$ die oben angegebene Bedeutung haben und der Piperazinylrest an den Kohlenstoffatomen 1–3 fach durch $C_1$–$C_4$-Alkyl, 2-Thienyl oder gegebenenfalls substituiertes Cyclohexyl oder Phenyl substituiert sein kann, mit Verbindungen der Formel (V)

$R^3X$ (V)

in welcher

$R^3$ die oben angegebene Bedeutung hat, jedoch nicht Wasserstoff sein kann, und

X Fluor, Chlor, Brom, Iod, Hydroxy, Acyloxy, Ethoxy, Phenoxy, 4-Nitrophenoxy bedeutet,

gegebenenfalls in Gegenwart von Säurebindern umsetzt (Methode B).

Man erhält erfindungsgemässe Verbindungen der Formel (I) auch, wenn man eine 7-(1-Piperazinyl)-3-chinoloncarbonsäure der Formel (IV), in welcher der Piperazinylrest an den Kohlenstoffatomen 1–3 fach durch $C_1$–$C_4$-Alkyl, 2-Thienyl oder gegebenenfalls substituiertes Cyclohexyl oder Phenyl substituiert sein kann, mit Michael-Acceptoren der Formel (VI),

$B–CH=CH_2$ (VI)

in der B für CN, $CO–R^6$ oder $COOR^7$ steht,

wobei $R^6$ für Methyl oder Ethyl und

$R^7$ für Methyl, Ethyl, n- oder i-Propyl steht,

umsetzt (Methode C).

Verwendet man bei der Umsetzung nach Methode A 2-Methylpiperazin und 6-Chlor-1-cyclopropyl- 7,8-difluor- 1,4-dihydro- 4-oxo-3-chinolincarbonsäure als Ausgangsstoffe, so kann der Reaktionsablauf durch das folgende Formelschema wiedergegeben werden:

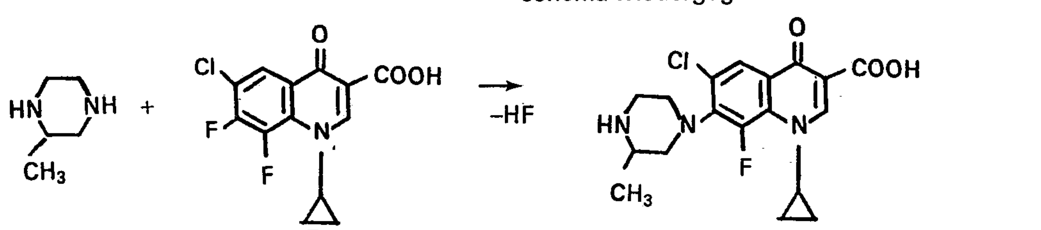


Verwendet man bei der Umsetzung nach Methode B Ethyliodid und 6-Chlor-1-cyclopropyl-8-fluor-1,4-dihydro-4-oxo-7-(1-piperazinyl)-3-chinolincarbonsäure als Ausgangsstoffe, so kann der Reaktionsablauf durch das folgende Formelschema wiedergegeben werden:

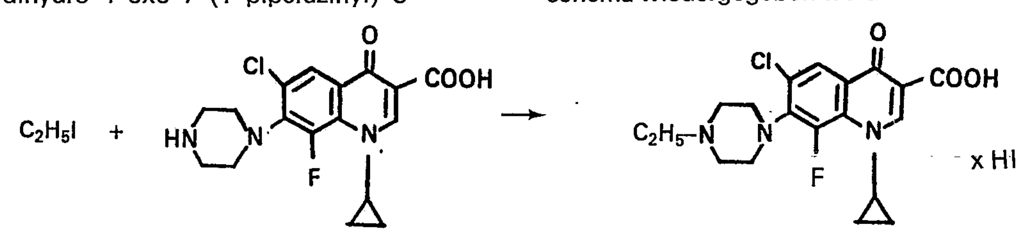

Verwendet man beispielsweise bei der Umsetzung von (IV) mit (V) nach Methode B 6-Chlor-1-cyclopropyl-8-fluor-1,4-dihydro-7-(3-methyl-1-piperazinyl)-4-oxo-3-chinolincarbonsäure und Ameisen-essigsäure-anhydrid als Ausgangsverbindungen, so kann der Reaktionsablauf durch das folgende Formelschema wiedergegeben werden:

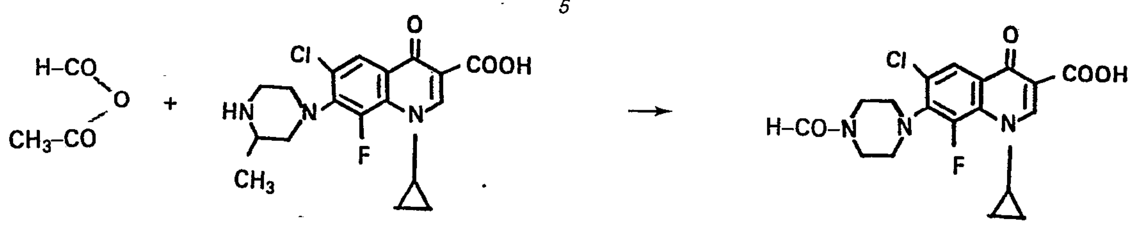

Verwendet man beispielsweise nach Methode C 6-Chlor-1-cyclopropyl-8-fluor-1,4-dihydro-4-oxo-7-(1-piperazinyl)-3-chinolincarbonsäure und Methylvinylketon als Ausgangsverbindungen, so kann der Reaktionsablauf durch das folgende Formelschema wiedergegeben werden:

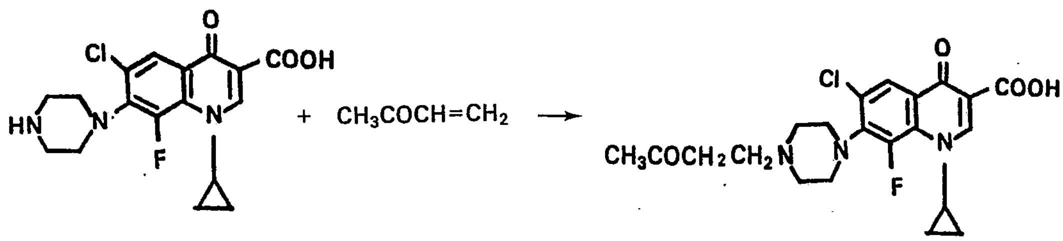


Die als Ausgangsstoffe nach Methode A verwendbaren 1-Cyclopropyl-6,7,8-trihalogen-1,4-dihydro-4-oxo-3-chinolincarbonsäuren der Formel (II) können gemäss folgendem Reaktionsschema hergestellt werden:

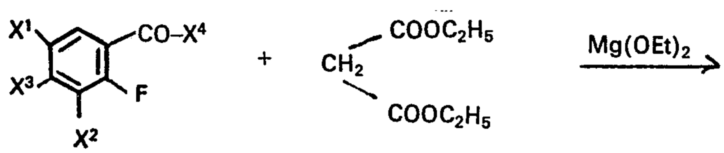


$X^4$=F oder Cl

(1) (2)

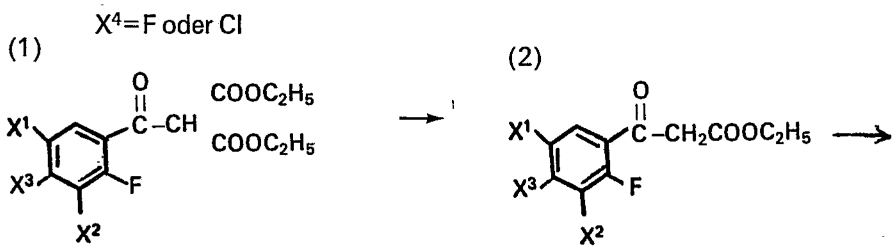

(3) (4)

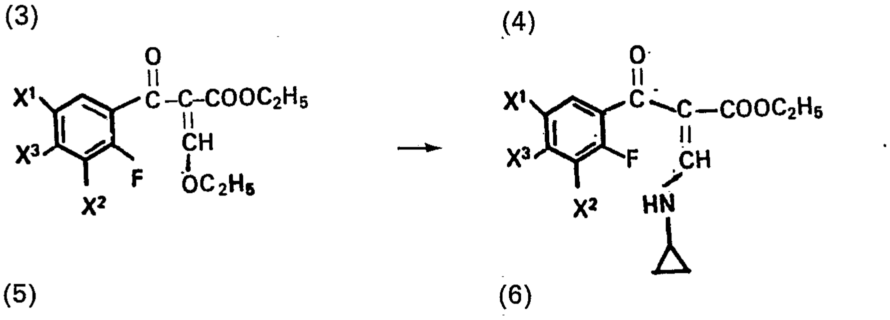

(5) (6)

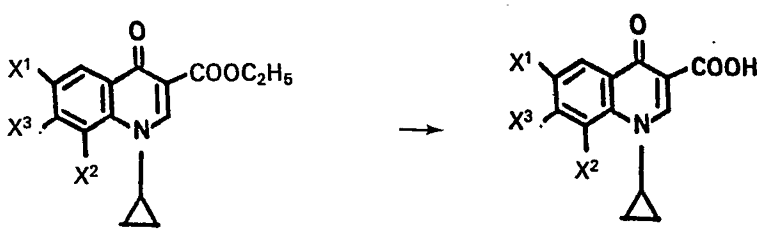

(7) (II)

Danach wird Malonsäurediethylester (2) in Gegenwart von Magnesiumethylat mit dem entsprechenden Benzoylfluorid bzw. -chlorid (1) zum Aroylmalonester (3) acyliert (Organicum, 3. Aufl. 1964, S. 438).

Durch partielle Verseifung und Decarboxylierung von (3) in wässrigem Medium mit katalytischen Mengen Schwefelsäure oder p-Toluolsulfonsäure erhält man in guter Ausbeute den Aroylessigsäureethylester (4), der mit Orthoameisensäure-triethylester/Acetanhydrid in den 2-(2,3,4,5- Tetrahalogenbenzoyl)- 3-ethoxy-acrylsäureethylester (5) übergeht. D: Umsetzung von (5) mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300 °C, bevorzugt 80 bis 180 °C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid, Natrium- oder Kaliumcarbonat und besonders bevorzugt Kalium- oder Natrium-fluorid in Betracht. Es kann vorteilhaft sein, einen Überschuss von 10 Mol-% an Base einzusetzen.

Die in dem letzten Schritt erfolgende Esterhydrolyse von (7) unter basischen oder sauren Bedingungen führt zu den 1-Cyclopropyl-6,7,8-trihalogen-1,4-dihydro-oxo-3-chinolincarbonsäuren (II).

Die als Ausgangsstoffe für diesen Syntheseweg verwendeten Benzoylhalogenide (1) werden wie folgt hergestellt:

3,5-Dichlor-2,4-difluor-benzoylfluorid (Siedepunkt 97°/20 mbar; $n^{20}_D$ = 1,5148) und 5-Chlor-2,3,4-trifluorbenzoylfluorid (Siedepunkt 68–70 °C/20 mbar; $n^{20}_D$ = 1,4764) erhält man nebeneinander beim Erhitzen von Tetrachlorbenzoylchlorid mit Kaliumfluorid im Sulfolan auf erhöhte Temperaturen:

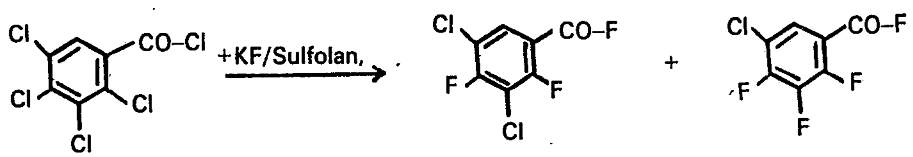


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94 °C/18 mbar; $n^{20}_D$ = 1,5164) umgesetzt wird:

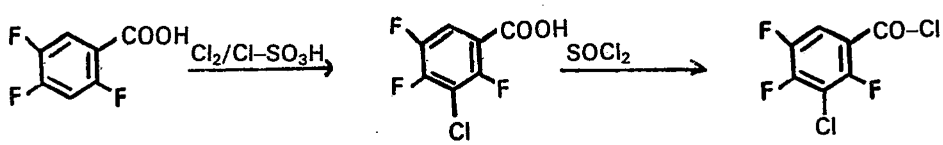


Die als Ausgangsstoffe verwendeten Amine (III) sind bekannt oder können nach literaturbekannten Verfahren erhalten werden [US 4 166 180, J. Med. Chem. 26, 1116 (1983). Durch katalytische Hydrierung werden aus den 2-Aryl-piperazinen die entsprechenden 2-Cyclohexylpiperazine erhalten; z.B.: 2-Cyclohexyl-piperazin (wachsartig, Schmelzpunkt 71–73 °C). Als Beispiele seien genannt:

Morpholin, Piperidin, Thiomorpholin, Pyrrolidin, Piperazin, N-Methylpiperazin, N-Ethylpiperazin, N-(2-Hydroxyethyl)-piperazin, N-Formylpiperazin, 2-Methylpiperazin, 1,2-Dimethylpiperazin, cis- und trans-2,5-Dimethylpiperazin, cis- und trans-2,6-Dimethylpiperazin, 2-Ethylpiperazin, 2-Propylpiperazin, 2-Isopropylpiperazin, 2-Isobutylpiperazin, 2-Piperazinon, 1-Methyl-2-piperazinon, 1-Ethyl-2-piperazinon, 2-Cyclohexylpiperazin, 2-Phenylpiperazin, 2-(4-Chlorphenyl)-piperazin, 2-(4-Fluorphenyl)-piperazin, 2-(4-Bromphenyl)-piperazin, 2-(4-Methylphenyl)-piperazin, 2-(4-Biphenyl)-piperazin, 2-(4-Methoxyphenyl)-piperazin, 2-(4-Benzyloxyphenyl)-piperazin, 2-(4-Hydroxyphenyl)-piperazin, 2-(4-Nitrophenyl)-piperazin, 2-(3-Nitrophenyl)-piperazin, 2-(4-Piperidinophenyl)-piperazin, 2-(3,4-Dimethoxyphenyl)-piperazin, 2-(3,4,5-Trimethoxyphenyl)-piperazin, 2-(3,4-dimethoxy-6-methyl)-piperazin, 2-(2-Thienyl)-piperazin, 3-Aminopyrrolidin.

Die als Ausgangsstoffe verwendeten Verbindungen der Formel (V) sind bekannt. Als Beispiele seien genannt:

Methyljodid, Methylbromid, Ethyljodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butylchlorid, n-Pentylchlorid, 3-Methylbutylchlorid, n-Hexylbromid, Ameisensäureessigsäureanhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid,Isobuttersäurechlorid, N-(tert.-Butoxycarbonyl)-glycin-4-nitrophenylester, N-(tert.-Butoxycarbonyl)-L-alanin-4-nitro-phenylester, N-(tert.-Butoxycarbonyl)-L-leucin-4- nitrophenylester, N-(tert.-Butoxycarbonyl)-L-valin-4-nitro-

phenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethylcarbamidsäurechlorid, Methansulfonsäurechlorid, Ethansulfonsäurechlorid, Propan-1-sulfonsäurechlorid, Ameisensäure.

Die erfindungsgemäss verwendbaren Verbindungen der Formel (VII)sind bekannt. Als Beispiele seien genannt:

Acrylnitril, Methylvinylketon, Acrylsäuremethylester, Acrylsäureethylester.

Die Umsetzung von (II) mit (III) gemäss Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]-octan (DABCO), 1,8-Diazabicyclo[5,4,0]-undec-7-en (DBU) oder überschüssiges Amin (III).

Die Reaktionstemperaturen können in einem grösseren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens setzt man auf 1 Mol der Carbonsäure (II) 1 bis 15 Mol, vorzugsweise 1 bis 6 Mol des Amins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazabicyclo[5,4,0]undec-7-en (DBU).

Die Reaktionstemperaturen können in einem grösseren Bereich variiert werden. Im allgemeinen arbeitet man zwischen etwa 20 und etwa 180 °C, vorzugsweise zwischen 40 und 110 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens gemäss Methode B setzt man auf 1 Mol der Verbindung (IV) 1 bis 4 Mol, vorzugsweise 1 bis 1,5 Mol der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem grösseren Bereich variiert werden. Im allgemeinen arbeitet man zwischen etwa 20 °C und etwa 150 °C, vorzugsweise zwischen 50 °C und 100 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens nach Methode C setzt man auf 1 Mol der Verbindung (IV) 1 bis 5 Mol, vorzugsweise 1 bis 2 Mol, der Verbindung (VI) ein.

Ausser den in den Beispielen aufgeführten Verbindungen seien als neue Wirkstoffe im einzelnen genannt:

6-Chlor-7-[3-(4-chlorphenyl)-1-piperazinyl]-1-cyclopropyl-8-fluor-1,4-dihydro-4-oxo-3-chinolincarbonsäure,

6-Chlor-1-cyclopropyl-8-fluor-7-[3-(4-fluorphenyl)-1-piperazinyl]-1,4-dihydro-4-oxo-3-chinolincarbonsäure,

7-[3-(4-Bromphenyl)-1-piperazinyl]-6-chlor-1-cyclopropyl-8-fluor-1,4-dihydro-4-oxo-3-chinolincarbonsäure,

6-Chlor-1-cyclopropyl-8-fluor-1,4-dihydro-7-[3-(4-methylphenyl)-1-piperazinyl]-4-oxo-3-chinolincarbonsäure,

7-[3-(4-Biphenylyl)-1-piperazinyl]-6-chlor-1-cyclopropyl-8-fluor-1,4-dihydro-4-oxo-3-chinolincarbonsäure,

6-Chlor-1-cyclopropyl-8-fluor-1,4-dihydro-7-[3-(4-methoxyphenyl)-1-piperazinyl]-4-oxo-3-chinolincarbonsäure,

6-Chlor-1-cyclopropyl-8-fluor-1,4-dihydro-7-[3-(4-hydroxyphenyl)-1-piperazinyl]-4-oxo-3-chinolincarbonsäure,

8-Chlor-1-cyclopropyl-6-fluor-1,4-dihydro-4-oxo-7-(3-phenyl-1-piperazinyl)-3-chinolincarbonsäure,

8-Chlor-1-cyclopropyl-6-fluor-1,4-dihydro-4-oxo-7-[(4-nitrophenyl)-1-piperazinyl]-3-chinolincarbonsäure,

8-Chlor-1-cyclopropyl-6-fluor-1,4-dihydro-4-oxo-7-[3-(4-piperidinophenyl)-1-piperazinyl]-3-chinolincarbonsäure,

8-Chlor-1-cyclopropyl-6-fluor-1,4-dihydro-4-oxo-7-[3-(3,4-dimethoxyphenyl)-1-piperazinyl]-3-chinolincarbonsäure,

8-Chlor-1-cyclopropyl-6-fluor-1,4-dihydro-4-oxo-7-[3-(3,4,5-trimethoxyphenyl)-1-piperazinyl]-3-chinolincarbonsäure,

8-Chlor-1-cyclopropyl-6-fluor-1,4-dihydro-4-oxo-7-[3-(2-thienyl)-1-piperazinyl]-3-chinolincarbonsäure,

8-Chlor-1-cyclopropyl-6-fluor-1,4-dihydro-4-oxo-7-piperidino-3-chinolincarbonsäure,

7-(3-Amino-1-pyrrolidinyl)-8-chlor-1-cyclopropyl-6-fluor-1,4-dihydro-4-oxo-3-chinolincarbonsäure,

6,8-Dichlor-1-cyclopropyl-1,4-dihydro-4-oxo-7-(1-piperazinyl)-3-chinolincarbonsäure,

7-(4-Acetyl-1-piperazinyl)-6,8-dichlor-1-cyclopropyl-1,4-dihydro-4-oxo-3-chinolincarbonsäure,

7-(4-Acetyl-1-piperazinyl)-6-chlor-1-cyclopropyl-8-fluor-1,4-dihydro-4-oxo-3-chinolincarbonsäure,

6-Chlor-1-cyclopropyl-8-fluor-1,4-dihydro-7-(4-isopropyl-1-piperazinyl)-4-oxo-3-chinolincarbonsäure,

6-Chlor-1-cyclopropyl-8-fluor-1,4-dihydro-4-oxo-7-morpholino-3-chinolincarbonsäure,

6-Chlor-1-cyclopropyl-8-fluor-1,4-dihydro-4-oxo-7-thiomorpholino-3-chinolincarbonsäure,

8-Chlor-1-cyclopropyl-7-(4-ethyl-3-oxo-1-piperazinyl)-6-fluor-1,4-dihydro-4-oxo-3-chinolincarbonsäure.

Die folgenden Beispiele erläutern die Erfindung:

Beispiel A

6-Chlor-1-cyclopropyl-7,8-difluor-1,4-dihydro-4-oxo-3-chinolincarbonsäure

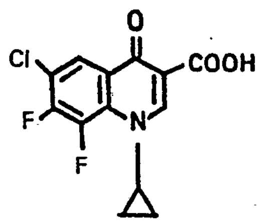

15,7 g (0,65 mol) Magnesiumspäne werden in 40 ml Ethanol und 2 ml Tetrachlormethan verrührt und nach begonnener Reaktion bei 50–60 °C tropfenweise mit 103 g (0,64 mol) Malonsäurediethylester in 80 ml Ethanol und 250 ml Toluol versetzt. Man rührt 1 Stunde bei dieser Temperatur nach, kühlt auf −5 bis −10°, tropft eine Lösung von 138 g (0,65 mol) 5-Chlor-2,3,4-benzoylfluorid in 63 ml Toluol zu, rührt noch 1 Stunde bei 0° und lässt über Nacht bei Raumtemperatur stehen. Danach wird noch 2 Stunden auf 40–50 °C erwärmt, abgekühlt und mit 250 ml Eiswasser und 38,5 ml konzentrierter Schwefelsäure versetzt. Die organische Phase wird abgetrennt, die wässrige Phase mit 2 mal 150 ml Toluol extrahiert, die vereinigten organischen Phasen mit gesättigter Natriumchlorid-Lösung gewaschen, mit Natriumsulfat getrocknet und eingeengt.

Der Rückstand wird mit 200 ml Wasser versetzt (vorteilhaft ist hier die Zugabe von 0,4 g 4-Toluolsulfonsäure) und zur Desethoxycarbonylierung 5 Stunden unter Rückfluss erhitzt. Man extrahiert mit 3 mal 200 ml Dichlormethan, wäscht mit gesättigter Natriumchlorid-Lösung, trocknet mit Natriumsulfat, engt ein und destilliert im Hochvakuum. Man erhält 103 g (56,5%) (5-Chlor-2,3,4-trifluor-benzoyl)-essigsäure-ethylester mit einem Siedepunkt von 110 °C/0,9 Torr (1,197 mbar).

103 g (0,37 mol) des erhaltenen Esters und 83 g (0,56 mol) Orthoameisensäuretriethylester werden mit 95 g Essigsäureanhydrid 2 Stunden auf 150–160 °C erhitzt und anschliessend bei 120–130 °C unter Normaldruck, danach im Hochvakuum eingeengt. Man erhält 115 g (92% der Theorie) 2-(5-Chlor-2,3,4-trifluor-benzoyl)-3-ethoxyacrylsäure-ethylester als Öl.

84,1 g (0,25 mol) dieser Verbindung werden in 170 ml Ethanol unter Eiskühlung tropfenweise mit 14,8 g (0,26 mol) Cyclopropylamin versetzt und 2 Stunden bei Raumtemperatur gerührt. Danach wird mit 170 ml Wasser verrührt, in Eis gekühlt, der ausgefallene Niederschlag abgesaugt, mit Wasser und wenig Methanol gewaschen und getrocknet. Es werden 47 g (54%) 2-(5-Chlor-2,3,4- trifluor- benzoyl)- 3-cyclopropylamino- acrylsäureethylester vom Schmelzpunkt 71–73 °C erhalten. Nach dem $^1$H-NMR-Spektrum liegt ein cis-trans-Gemisch vor.

47 g (0,14 mol) dieser Verbindung werden in 230 ml Dimethylformamid mit 9,7 g (0,23 mol) Natriumfluorid 2 Stunden auf 160–170 °C erhitzt. Das Reaktionsgemisch wird in 400 ml Eiswasser eingegossen, der Niederschlag abgesaugt, mit Wasser gewaschen und getrocknet. Man isoliert 44 g (99%) 6-Chlor-1-cyclopropyl-7,8-difluor-1,4-dihydro-4-oxo-3-chinolincarbonsäure-ethylester vom Schmelzpunkt 169–172 °C.

44 g (0,13 mol) des Chinoloncarbonsäureesters werden in 300 ml Eisessig und 179 ml Wasser mit 33 ml konzentrierter Schwefelsäure versetzt und 2 Stunden auf 150 °C erhitzt. Das Reaktionsgemisch wird in 400 ml Eiswasser eingerührt, der Niederschlag abgesaugt, mit Wasser gewaschen und getrocknet. Es werden 37 g (95% der Theorie) 6-Chlor-1-cyclopropyl-7,8-difluor-1,4-dihydro-4-oxo-3-chinolincarbonsäure mit einem Schmelzpunkt von 200–204 °C isoliert.

Beispiel B

8-Chlor- 1-cyclopropyl- 6,7-difluor-1,4-dihydro-4-oxo-3-chinolincarbonsäure

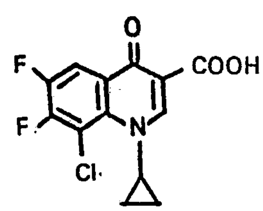

Man setzt 3-Chlor-2,4,5-trifluor-benzoylchlorid analog Beispiel A um, wobei folgende Stufen durchlaufen werden:

(3-Chlor- 2,4,5-trifluor-benzoyl)- essigsäure-ethylester als Enol (Ausbeute: 42%, Schmelzpunkt 72–75 °C),

2-(3-Chlor-2,4,5-trifluor-benzoyl)-3-ethoxyacrylsäure-ethylester (Rohausbeute: 95% Öl),

2-(3-Chlor-2,4,5-trifluor-benzoyl)-3-cyclopropylaminoacrylsäureethylester (Ausbeute: 67%, Schmelzpunkt 78–80 °C),

8-Chlor-1-cyclopropyl-6,7-difluor-1,4-dihydro-4-oxo-3-chinolincarbonsäure-ethylester (Ausbeute: 85%, Schmelzpunkt 154–157 °C),

8-Chlor-1-cyclopropyl-6,7-difluor-1,4-dihydro-4-oxo-3-chinolincarbonsäure (Ausbeute: 97,6%, Schmelzpunkt 189–192 °C).

Beispiel C

6,8-Dichlor-1-cyclopropyl-7-fluor-1,4-dihydro-4-oxo-3-chinolincarbonsäure

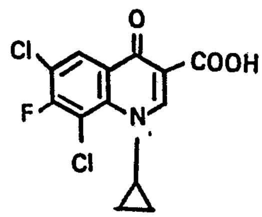

Man setzt 3,5-Dichlor-2,4-difluor-benzoylfluorid analog Beispiel A um, wobei folgende Stufen durchlaufen werden:

(3,5-Dichlor-2,4-difluor-benzoyl)-essigsäureethylester (Ausbeute: 43%, Siedepunkt 133 °C/2,5 Torr (3,325 mbar),

2-(3,5-Dichlor-2,4-difluor-benzoyl)-3-ethoxy-acrylsäureethylester (Rohausbeute: 91% Öl),

2-(3,5-Dichlor-2,4-difluor-benzoyl)-3-cyclopropylaminoacrylsäureethylester (Ausbeute: 96%, Schmelzpunkt 71–74 °C),

6,8-Dichlor-1-cyclopropyl-7-fluor-1,4-dihydro-4-oxo-3-chinolincarbonsäure-ethylester (Ausbeute: 97%, Schmelzpunkt 215–217 °C unter Zersetzung),

6,8-Dichlor-1-cyclopropyl-7-fluor-1,4-dihydro-4-oxo-3-chinolincarbonsäure (Ausbeute: 93%, Schmelzpunkt 204–206 °C).

Beispiel 1

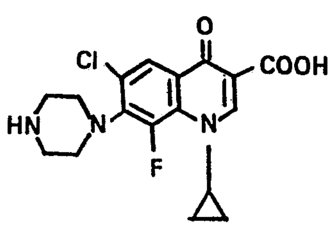

12 g (40 mmol) des Produktes aus Beispiel A werden in 100 ml Pyridin mit 17,2 g (0,2 mol) Piperazin 5 Stunden unter Rückfluss erhitzt. Das Gemisch wird im Vakuum eingeengt, mit 120 ml Wasser verrührt und mit 2 n Salzsäure auf pH 5 eingestellt. Der Niederschlag wird abgesaugt, mit Wasser und Methanol gewaschen, in 80 ml Methanol aufgekocht und getrocknet. Man erhält 12,3 g (84% der Theorie) 6-Chlor-1-cyclopropyl-8-fluor- 1,4-dihydro- 4-oxo-7- (1-piperazinyl)-3-chinolincarbonsäure vom Schmelzpunkt 295–298 °C (unter Zersetzung).

Analog Beispiel 1 erhält man folgende in 7-Stellung substituierte 6-Chlor-1-cyclopropyl-8-fluor- 1,4-dihydro- 4-oxo- 3-chinolincarbonsäuren:

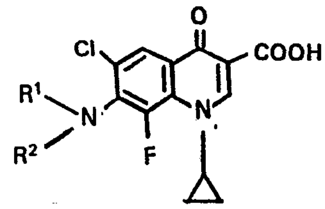

| Beispiel | 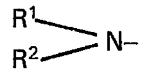 | Schmelzpunkt |
|---|---|---|
| 2 | 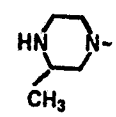 | 258–282 °C (Zersetzung) |
| 3 | 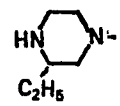 | 191–195 °C (Zersetzung) |
| 4 | 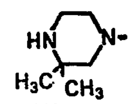 | ab ~ 274 °C (Zersetzung) |
| 5 | 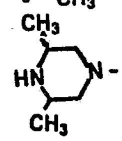 | 255–261 °C (Zersetzung) |
| 6 | 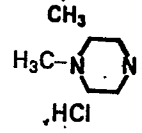 | > 320 °C (Zersetzung) |
| 7 | 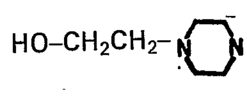 | 276–280 °C (Zersetzung) |
| 8 | 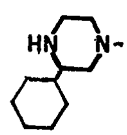 | ab ~ 190 °C (Zersetzung) |
| 9 | 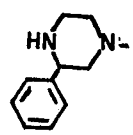 | 154–158 °C |

Beispiel 10

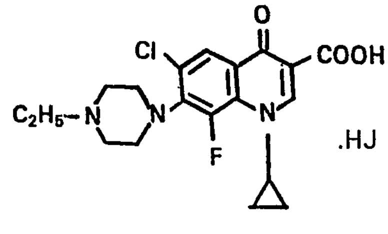

.HJ

1,83 g (5 mmol) des Produktes aus Beispiel 1 werden in 20 ml Dimethylformamid mit 1,6 g Ethyljodid und 1 g Triethylamin 3 Stunden auf 80 °C erhitzt. Das Reaktionsgemisch wird im Vakuum eingeengt, der Rückstand mit 20 ml Wasser verrührt und aus Methanol umkristallisiert. Man erhält 0,4 g (15% der Theorie) 6-Chlor-1-cyclopropyl- 7-(4-ethyl-1-piperazinyl)- 8-fluor-1,4-dihydro-4-oxo-3-chinolincarbonsäure vom Schmelzpunkt 237–242 °C (unter Zersetzung).

Beispiel 11

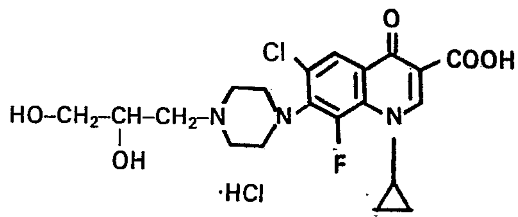

3,65 g (0,01 mmol) des Produktes aus Beispiel 1 werden in 150 ml Ethanol und 30 ml Wasser suspendiert, die Suspension wird mit Essigsäure auf pH 4,6 eingestellt und anschliessend bei Raumtemperatur mit 3,4 g (0,02 mol) 2,3-cyclohexylidenglycerinaldehyd und portionsweise mit 950 mg Natriumcyanoborhydrid versetzt. Man rührt über Nacht bei Raumtemperatur, stellt mit Natriumhydrogencarbonat auf pH 8, extrahiert mit Dichlormethan und engt den Extrakt ein.

Der Rückstand wird in 25 ml Ethanol und 25 ml Wasser mit 3 ml Konzentrierter Salzsäure versetzt und 6 Stunden unter Rückfluss erhitzt. Das Gemisch wird eingeengt, in Wasser gelöst, mit Dichlormethan extrahiert, nochmals eingeengt, der Rückstand mit Ethanol verrührt und getrocknet. Man erhält 1,3 g 6-Chlor-1-cyclopropyl-8-fluor-1,4-dihydro-7-[4-(2,3-dihydroxypropyl)-1-piperazinyl]-4-oxo-3-chinolincarbonsäure-Hydrochlorid vom Schmelzpunkt 263–266 °C (unter Zersetzung).

Beispiel 12

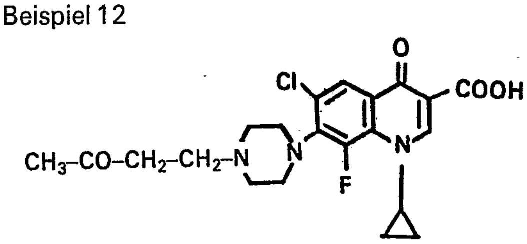

1,83 g 65 mmol) des Produnktes aus Beispiel 1 und 1,95 g (28 mmol) Methylvinylketon werden in 25 ml Ethanol 6 Stunden unter Rückfluss erhitzt, der Niederschlag abgesaugt und aus Glykolmonomethylether umkristallisiert. Es werden 1 g (46% der Theorie) 6-Chlor-1-cyclopropyl-8-fluor- 1,4-dihydro-4-oxo-7- [4-(3-oxo-butyl)-1-piperazinyl]-3-chinolincarbonsäure vom Schmelzpunkt 187–190 °C (unter Zersetzung) erhalten.

Beispiel 13

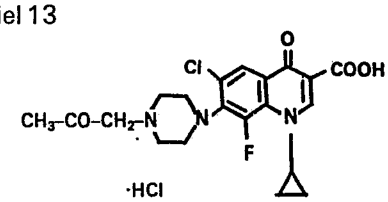

1,83 g (5 mmol) des Produktes aus Beispiel 1 werden in 25 ml Ethanol mit 1,95 g (20 mmol) Chloraceton 6 Stunden unter Rückfluss erhitzt. Man kühlt die Suspension ab, saugt sen Niederschlag ab, wäscht gut mit Ethanol und trocknet im Vakuum, wobei 1 g (44% der Theorie) 6-Chlor-1-cyclopropyl- 8-fluor- 1,4-dihydro-4-oxo-7-[4-(2-oxo-propyl)- 1-piperazinyl]-3-chinolincarbonsäure-Hydrochlorid vom Schmelzpunkt ~ 320 °C (unter Zersetzung) erhalten werden.

Beispiel 14

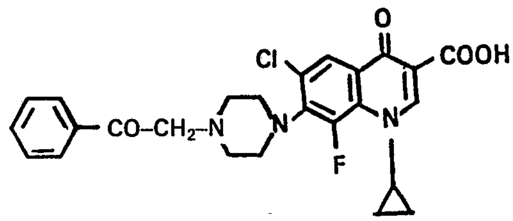

3,66 g (0,01 mol) des Produktes aus Beispiel 1 werden in 50 ml Dimethylformamid mit 2,2 g ω-Chloracetophenon und 2,2 g Triethylamin 10 Stunden auf 60° erhitzt. Das Reaktionsgemisch wird im Vakuum eingeengt, mit 30 ml Wasser verrührt, der Niederschlag abgesaugt, mit Wasser gewaschen und aus Aceton umkristallisiert. Man erhält 1,2 g (25% der Theorie) 6-Chlor-1-cyclopropyl-8-fluor-1,4-dihydro- 4-oxo-7-[4-(2-oxo-2-phenyl-ethyl)-1-piperazinyl]-3-chinolincarbonsäure vom Schmelzpunkt 175–179 °C (unter Zersetzung).

Beispiel 15

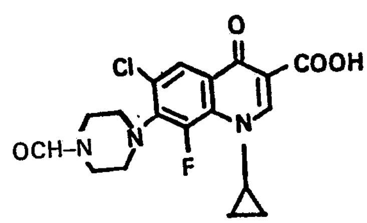

1,5 g (4 mmol) des Produktes aus Beispiel 1 werden in einer Mischung aus 10 ml Dioxan und 170 mg Natriumhydroxid in 2,5 ml Wasser gelöst und dann gleichzeitig mit einer Lösung von 0,7 g Ameisensäureessigsäureanhyrid in 5 ml Dioxan und einer Lösung von 340 mg Nartiumhydroxid in 5 ml Wasser versetzt. Man rührt 2 Stunden bei Raumtemperatur, verdünnt mit 30 ml Wasser, saugt den Niederschlag ab, wäscht mit Wasser und Methanol und kristallisiert aus Glykolmonomethylether um. Es werden 0,6 g (38%) 6-Chlor-1-cyclopropyl- 8-fluor-7- (4-formyl-1-piperazinyl)-1,4-dihydro- 4-oxo- 3-chinolincarbonsäure vom Schmelzpunkt 277–278 °C (unter Zersetzung) erhalten.

Beispiel 16

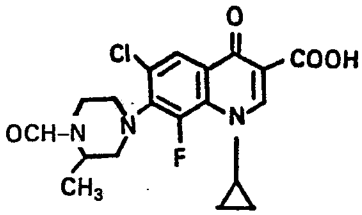

Setzt man analog Beispiel 15 das Produkt aus Beispiel 2 um, dann erhält man 6-Chlor-1-cyclopropyl-8-fluor-7-(4-formyl-3-methyl-1-piperazinyl)-1,4-dihydro- 4-oxo- 3-chinolincarbonsäure vom Schmelzpunkt 280–282 °C (unter Zersetzung).

Beispiel 17

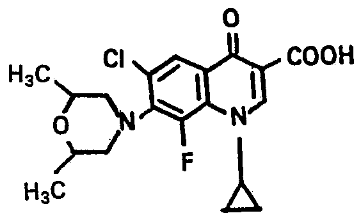

3 g (10 mmol) des Produktes aus Beispiel A werden in 35 ml Dimethylsulfoxid mit 1,2 g (10 mmol) 2,6-Dimethylmorpholin und 2,2 g (20 mmol) Diazabicyclo [2.2.2] octan 5 Stunden auf 140° erhitzt. Man engt im Hochvakuum ein, verrührt mit 30 ml Wasser, stellt mit 2 n Salzsäure auf pH 6, saugt den Niederschlag ab und kristallisiert aus Glykolmonomethylether um. Es werden 1,6 g (41% der Theorie) 6-Chlor-1-cyclopropyl-8-fluor- 1,4-dihydro- 7-(2,6-dimethyl- 4-morpholinyl)- 4-oxo- 3-chinolincarbonsäure vom Schmelzpunkt 258–261 °C (unter Zersetzung) erhalten.

Beispiel 18

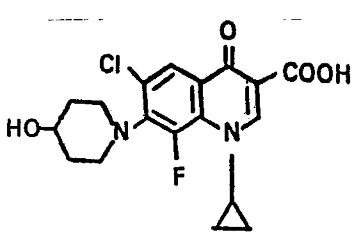

Setzt man analog Beispiel 17 das Produkt aus Beispiel A mit 4-Hydroxypiperperidin um, dann erhält man 6-Chlor-1-cyclopropyl-8-fluor-1,4-dihydro-7- (4-hydroxy-1-piperdinyl)- 4-oxo-3-chinolincarbonsäure vom Schmelzpunkt 226–231 °C (unter Zersetzung).

Beispiel 19

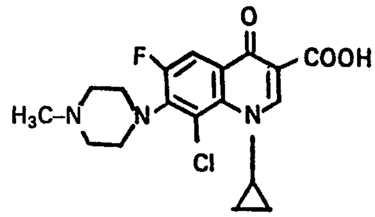

3 g (0,01 mol) des Produktes aus Beispiel B werden in 25 ml Pyridin mit 4 g (0,04 mol) 1-Methyl-piperazin 5 Stunden unter Rückfluss erhitzt. Man en engt im Vakuum ein, versetzt mit 20 ml Wasser, stellt mit 2 n Salzsäure auf pH 5 und kristallisiert den ausgefallenen Niederschlag aus Methanol um. Es werden 0,6 g (16% der Theorie) 8-Chlor-1-cyclopropyl-6-fluor-1,4-dihydro-7-(4-methyl-1-peperazinyl)-4-oxo-3-chinolincarbonsäure vom Schmelzpunkt 293–297 °C (unter Zersetzung) erhalten.

Beispiel 20

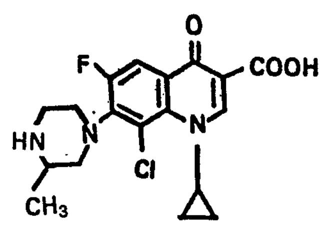

Analog Beispiel 19 erhält man mit 2-Methylpiperazin- 8-Chlor-1-cyclopropyl-6-fluor-1,4-dihydro-7-(3-methyl-1-piperazinyl)-4-oxo-3-chinolincarbonsäure vom Schmelzpunkt 318–325 °C (unter Zersetzung).

Beispiel 21

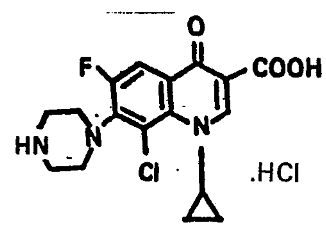

Man setzt analog Beispiel 19 das Produkt aus Beispiel B mit Piperazin 1,5 Stunden unter Rückfluss um und behandelt das Reaktionsgemisch mit Salzsäure, wobei 8-Chlor-1-cyclopropyl-6-fluor-1,4-dihydro-7-(1-piperazinyl)-3-chinolincarbonsäure-Hydrochlorid mit einem Zersetzungspunkt oberhalb 330 °C erhalten wird.

Beispiel 22

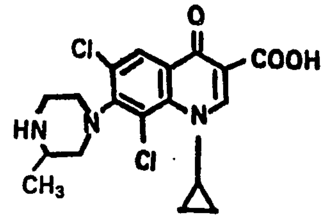

Setzt man das Produkt aus Beispiel C analog Beispiel 19 mit 2-Methyl-piperazin um, dann erhält man 6,8-Dichlor-1-cyclopropyl-1,4-dihydro-7-(3-methyl-1-piperazinyl)-4-oxo-3-chinolincarbonsäure vom Schmelzpunkt 288–291 °C (unter Zersetzung).

Beispiel 23

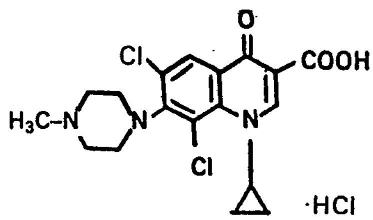

3,2 g (0,01 mol) des Produktes aus Beipiel C werden mit 4 g (0,04 mol) 1-Methyl-piperazin 3 Tage auf 80° erhitzt, das Reaktionsgemisch im Vakuum eingeengt, der Rückstand in etwas Wasser aufgenommen und mit 2 n Salzsäure auf pH 7 eingestellt. Beim Stehen in Eis erfolgt Kristallisation. Der Niederschlag wurde abgesaugt und aus Wasser unter Zusatz von etwas Salzsäure umkristallisiert. Man erhält 0,6 g 6,8-Dichlor-1-cyclopropyl-1,4-dihydro-7-(4-methyl-1-piperazinyl)-4-oxo-3-chinolincarbonsäure vom Schmelzpunkt > 300 °C.

Beispiel 24

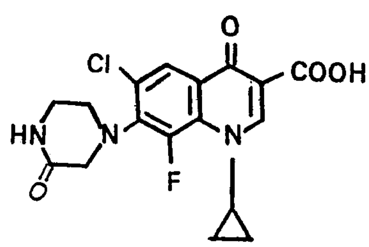

3 g (10 mmol) des Produktes aus Beispiel 1 werden in 25 ml Dimethylsulfoxid mit 1,8 g (18 mmol) 2-Piperazinon und 2,2 g (20 mmol)-Diazabicyclo [2.2.2] octan 2 Stunden auf 130 °C erhitzt. Die Suspension wird mit 2 n Salzsäure auf pH 5 eingestellt, mit 25 ml Wasser versetzt und der Niederschlag abgesaugt, mit 20 ml Methanol ausgekocht und getrocknet. Es wedern 1,5 g (39% der Theorie) 6-Chlor-1-cyclopropyl-8-fluor-1,4-dihydro- 4-oxo-7-(3-oxo-1-piperazinyl)-3-chinolincarbonsäure vom Schmelzpunkt 288–291 °C (unter Zersetzung) erhalten.

Beispiel für eine erfindungsgemässe Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon)unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)cellulose 15 cp | 6,0 mg |
| Macrogol 4000 rec. INN (Polyethylenglykole DAB) | 2,0 mg |
| Titan-(IV)-oxid | 2,0 mg |
| | 10,0 mg |

Die erfindungsgemässen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracyline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemässen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemässen Verbindungen gegen Bakterein und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. Epedermidis, (Staph. = Staphylococcus); Lactobacteriaceae, wie Streptokokken, z.B. Streptococcus pyogenes, α– bzw. 8-hämolysierende Streptokokken, nicht-γ-hämolysierende Streptokokken, Enterokokken und Diplococcus pneumoniae (Pneumokokken) (Str. = Streptococcus); Enterobacteriaceae, wie Escherichiae-Bakterien der Coli-Gruppe: Escherichia-Bakterien, z.B. Escherichia coli, Enterobacter-Bakterien, z.B. E. aerogenes, E. clocae (E.=Enterobacter), Klebsiella-Bakterien, z.B. K. pneumoniae (K.=Klebsiella), Serratia, z.B. Serratia marcescens, Proteae-Bakterien der Proteus-Gruppe: Proteus, z.B. Pr. vulgaris, Pr.morganii, Pr.rettgeri, Pr.mirabilis (Pr.=Proteus); Pseudomonadaceae, wie Pseudomonas-Bakterien, z.B. Ps, aeruginosa(Ps.=Pseudomonas); Bacteroidaceae, wie Bacteroides-Bakterien, z.B. Bacteroides fragilis, Mykroplasmen, z.B. Mykoplasma pneumoniae ausserdem Mykobakterien, z.ß. Mykobacterium tuberculosis, Mycobakterium leprae und atypische Mykobakterien.

Die obige Aufzählung von Erregern ist lediglich beispielhaft und keineswegs beschränkend aufzufassen. Als Krankheiten, die durch die erfindungsgemässen Verbindungen verhindert, gebessert und/oder geheilt werden können, seien beispielsweise genannt:

Erkrankungen der Atmungswege und des Rachenraumes;

Otitis, Pharyngitis; Pneumonie; Peritonitis; Pyelonephritis; Cystitis; Endocarditis; Systeminfektionen; Bronchitis; Arthritis; lokale Infektionen, septische Erkrankungen.

Zur vorliegenden Erfindung gehören pharmazeutische Zubereitungen, die neben nicht-toxischen, inerten pharmazeutisch geeigneten Trägerstoffen einen oder mehrere erfindungsgemässe Verbindungen enthalten oder die aus einem oder mehreren erfindungsgemässen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen , einer halben oder einem Drittel oder einem Viertel einer Tagesdosis entspricht.

Unter nicht-toxischen, inerten pharmazeutisch geeigneten Trägerstoffen sind feste, halbfeste oder füssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll– und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonosterarat, (h)Adsorptionsmittel, Z.B. Kaolin und Bentonit und(i) Gleitmittel, z.B. Talkum, Calcium- und Magnesiumstearat und feste Polyethylenglykole oder Gemische der unter (a) bis (i) aufgeführten Stoffe.

Die Tabletten Dragees, Kapseln, Pillen und Granulate können mit den üblichen, gegebenenfalls Opakisierungsmittel enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffen enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlen Wasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z,B. Wasser, Ethylalkohol, Propylenalkohol, Suspendiermittel, z.B. ethoxylierte Isostearylalkohole, Polyoxyethylensorbit- und Sorbitan-Ester, mikrokristalline Cellulose, Aliminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süssmittel, Z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise

von etwa 0,5 bis 95 Gew.-%, der Gesamtmischung vorhanden sein.

Die oben aufgeführten pharmazeutischen Zubereitungen können ausser den erfindungsgemässen Verbindungen auch weitere pharmzeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 0,5 bis 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemässen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrung seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In den nachstehenden Tabelle sind die MHK-Werte einiger der erfindungsgemässen Verbindungen angegeben.

Als Vergleich wurden die entsprechenden MHK-Werte der aus J. Med. Chem. 23, 1358 (1980) bekannten 1-Ethyl-6-fluor-1,4-dihydro-4-oxo-7- (1-piperazinyl)-3-chinolincarbonsäure («Norfloxacin») angegeben, wobei sich zeigt, dass die erfindungsgemässen Verbindungen der bekannten Verbindung überlegen sind.

MIC (mcg/ml)

| Stamm | Beispiel Nr. | 1 | 2 | 3 | 5 | 6 | 7 | 8 | 10 |
|---|---|---|---|---|---|---|---|---|---|
| E.coli Neumann | | ≤ 0,015 | ≤ 0,015 | ≤ 0,015 | ≤ 0,015 | ≤ 0,015 | ≤ 0,015 | – | ≤ 0,015 |
| T7 | | ≤ 0,015 | ≤ 0,015 | ≤ 0,015 | ≤ 0,015 | ≤ 0,015 | ≤ 0,015 | ≤ 0,015 | 0,025 |
| 445/7 | | 4 | 8 | 8 | 8 | 4 | 16 | – | 16 |
| Klebsiella 63 | | ≤ 0,015 | 0,03 | 0,03 | 0,03 | 0,015 | 0,06 | – | 0,125 |
| 6179 | | 0,03 | 0,03 | ≤0,015 | 0,03 | 0,015 | 0,06 | – | 0,06 |
| Proteus 1017 | | ≤ 0,015 | – | – | – | 0,06 | 0,125 | – | – |
| Providencia 12012 | | ≤ 0,015 | 0,03 | – | – | 0,03 | – | – | – |
| 12052 | | 8 | 16 | 16 | 32 | 4 | 16 | – | – |
| Staph. FK 422 | | 0,25 | 0,25 | 0,25 | 0,25 | 0,025 | 0,5 | 0,06 | – |
| 1756 | | 0,25 | 0,5 | 0,25 | 0,5 | 0,5 | 0,5 | 0,06 | – |
| 133 | | 0,25 | 0,25 | 0,25 | 0,5 | 0,25 | 0,25 | 0,06 | – |
| Pseudom. Ellsworth | | 0,06 | – | – | – | – | – | – | – |

Agardilutionstest / Isosensitestmedium

MIC (mcg/ml)

| Stamm | Beispiel Nr. | 17 | 12 | 13 | 14 | 19 | 20 | 21 | 22 | Norfloxacin |
|---|---|---|---|---|---|---|---|---|---|---|
| E.coli Neumann | | – | ≤ 0,015 | ≤ 0,015 | ≤ 0,015 | ≤ 0,015 | ≤ 0,015 | ≤ 0,015 | ≤ 0,015 | 0,06 |
| | T7 | – | ≤ 0,015 | ≤ 0,015 | ≤ 0,015 | ≤ 0,015 | ≤ 0,015 | ≤ 0,015 | ≤ 0,015 | 0,03 |
| | 455/7 | – | 8 | 8 | 2 | 1 | 1 | 1 | 2 | 16 |
| Klebsiella | 63 | – | ≤ 0,015 | ≤ 0,015 | 0,06 | 0,03 | ≤0,015 | 0,06 | 0,03 | 0,125 |
| | 6179 | – | 0,06 | 0,06 | 0,25 | 0,03 | 0,03 | 0,03 | 0,06 | 0,25 |
| Proteus | 1017 | – | 0,03 | ≤ 0,015 | 0,125 | 0,03 | ≤ 0,015 | 0,03 | 0,06 | 0,03 |
| Providencia | 12012 | – | ≤ 0,015 | ≤ 0,015 | 0,06 | 0,03 | 0,03 | 0,06 | 0,06 | 0,03 |
| | 12052 | – | 16 | 16 | 16 | 4 | 4 | 1 | 2 | 64 |
| Staph.FK | 422 | ≤ 0,015 | 0,25 | 0,25 | – | 0,06 | 0,06 | 0,125 | 0,125 | 0,5 |
| | 1756 | ≤ 0,015 | 0,25 | 0,25 | – | 0,06 | 0,06 | 0,125 | 0,125 | 1 |
| | 133 | – | 0,25 | 0,25 | – | 0,06 | 0,06 | 0,06 | 0,125 | 0,5 |
| Pseud.Ellsworth | | – | 0,125 | 0,125 | – | 0,125 | 0,25 | – | – | 0,125 |

Agardilutionstest / Isosensitestmedium

## Patentansprüche

1. 7-Amino-1-cyclopropyl-6,8-dihalogen-1,4-diahydro-4-oxo-3-chinolincarbonsäuren der Formel (I)

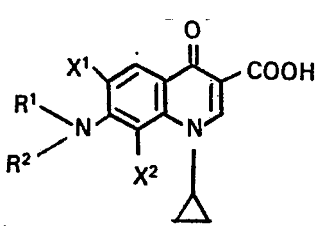

(I)

in welcher

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatom, an das sie gebunden sind, einen 5– oder 6–gliedrigen heterocyclischen Ring bilden, der als Ringglied zusätzlich die Atome oder Gruppen –O–, –S–, –SO–, $SO_2$, N–$R^3$ oder –CO–N–$R^3$ enthalten kann und der gegebenenfalls an den Kohlenstoffatomen ein- bis dreifach durch $C_1$–$C_4$-Alkyl, gegebenenfalls durch Chlor, Fluor, Brom, Methyl, Phenyl, Hydroxy, Methoxy, Benzyloxy, Nitro oder

Piperidino ein– bis dreifach substituiertes Phenyl oder Cyclohexyl, 2-Thienyl, Hydroxy, Alkoxy mit 1 bis 3 Kohlenstoffatomen, Amino, Methylamino oder Ethylamino substituiert sein kann, wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine oder zwei Hydroxy-, Alkoxy-, Alkylamino- oder Dialkylamino-gruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein– oder zweifach substituierten Phenacylrest, einen Oxoalkylrest mit bis zu 6 Kohlenstoffatomen steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen, wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino oder Alkylamino oder Dialkylamino mit 1 bis 5 Kohlenstoffatomen im Alkylteil und

$R^5$ geradkettiges oder verzweigtes Alkyl mit 1 bis 3 Kohlenstoffatomen darstellt, und deren pharmazeutisch verwendbaren Hydrate, Säureadditionssalze, Alkali-, Erdalkali- und Guanidiniumsalze.

2. Verbindungen der Formel (I) in Anspruch 1, in denen

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatom, an das sie gebunden sind, einen 5- oder 6-gliedrigen heterocyclischen Ring bilden können, der als Ringglied zusätzlich die Atome oder Gruppen –O–, –S–, $-SO^2-$, $-N-R^3$ oder $-CO-\overset{|}{N}-R^3$ enthalten kann und der gegebenenfalls an den Kohlenstoffatomen ein- oder zweifach durch $C_1$–$C_3$-Alkyl, Cycolhexyl, gegebenenfalls durch Chlor, Fluor, Brom, Methyl, Phenyl, Hydroxy, Methoxy, Benzyloxy, Nitro oder Piperidino ein oder zweifach substituiertes Phenyl, 2-Thienyl, Hydroxy, Amino oder Methylamino substituiert sein kann, wobei

$R^3$ für Wasserstoff, eine verzweigte oder unverzweigte Alkyl-, Alkenyl- oder Alkinylgruppe mit 1 bis 4 Kohlenstoffatomen, die gegebenenfalls durch eine oder zwei Hydroxygruppen substituiert sein kann, einen Phenylacylrest, einen Oxoalkylrest mit bis zu 5 Kohlenstoffatomen sowie für einen Rest $COR^4$ steht, wobei

$R^4$ Wasserstoff, geradkettiges oder verzweigtes Alkyl mit 1–3 Kohlenstoffatomen Alkoxy mit 1–3 Kohlenstoffatomen, Amino, Alkylamino oder Dialkylamino mit jweils 1–3 Kohlenstoffatomen im Alkylteil bedeutet.

3. Verbindungen der Formel (I) in Anspruch 1, in denen

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatom, an das sie gebunfen sind, einen 5– oder 6–gliedrigen heterocyclischen Ring bilden können der als Ringglied zusätzlich ein Sauerstoffatom oder die Gruppen $N-R^3$ oder $-CO-\overset{|}{N}-R^3$ enthalten kann und der gegebenenfalls an den Kohlenstoffatomen ein- oder zweifach durch $C_1$–$C_2$-Alkyl, Cyclohexyl, gegebenenfalls durch Chlor, Fluor, Methyl. Phenyl, Hydroxy, Methoxy, Benzyloxy, Nitro oder Piperidino substituiertes Phenyl, 2-Thienyl, oder Hydroxy substituiert sein kann, wobei

$R^3$ für Wasserstoff, eine verzweigte oder unverzweigte Alkylgruppe mit 1 bis 3 Kohlenstoffatomen die gegebenenfalls durch eine oder zwei Hydroxygruppe(n) substituiert sein kann, einen Phenylacylrest, einen Oxoalkylrest mit ein bis zu 4 Kohlenstoffatomen sowie für einen Rest $COR^4$ steht, wobei

$R^4$ Wasserstoff oder Alkyl mit ein oder zwei Kohlenstoffatomen bedeutet.

4. 7-(3-Amino-1-pyrrolidinyl)-8-chlor-1-cyclopropyl-6-fluor-1,4-dihydro-4-oxo-3-chinolincarbonsäure.

5. 6-Chlor-1-cyclopropyl-8-fluor-1,4-dihydro-4-oxo-7-(1-piperazinyl)-3-chinolincarbonsäure.

6. 6-Chlor-1-cyclopropyl-7,8-difluor-1,4-dihydro-4-oxo-3-chinolincarbonsäure.

7. 8-Chlor-1-cyclopropyl-6,7-difluor-1,4-dihydro-4-oxo-3-chinolincarbonsäure.

8. 6,8-Dichlor-1-cyclopropyl-7-fluor-1,4-dihydro-4-oxo-3-chinolincarbonsäure.

9. 7-Amino-1-cyclopropyl-6,8-dihalogen-1,4-dihydro-4-oxo-3-chinolincarbonsäuren der Formel (I) gemäss Anspruch 1 zur Verwendung bei der Bekämpfung von Erkrankungen.

10. Verbindungen der Formel (I) gemäss Anspruch 2 zur Verwendung bei der Bekämpfung von Erkrankungen.

11. Verbindungen der Formel (I) gemäss Anspruch 3 zur Verwendung bei der Bekämpfung von Erkrankungen.

12. Arzneimittel, gekennzeichnet durch einen Gehalt an 7-Amino-1-cyclopropyl-6,8-dihalogen-1,4-dihydro-4-oxo-3-chinolincarbonsäuren der Formel (I) gemäss Anspruch 1

13. Verfahren zur Herstellung von 7-Amino-1-cyclopropyl- 6,8-dihalogen- 1,4-dihydro-4-oxo-3-chinolincarbonsäuren der Formel (I)

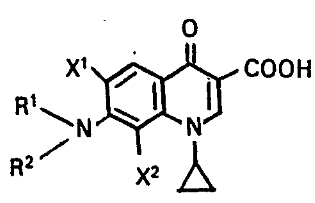

(I)

in welcher

$X^1$ und $X^2$ gleich oder verschieden sein können und für Chlor oder Fluor stehen, jedoch nicht gleichzeitig Fluor sein können und

$R^1$ und $R^2$ gemeinsam mit dem Stickstoffatomen, an das sie gebunden sind, einen 5– oder 6–gliedrigen heterocyclischen Ring bilden, der als Ringglied zusätzliclich die Atome oder Gruppen

–O–, –S–, –SO–, $-SO_2$, N–$R^3$ oder –CO–N–$R^3$ enthalten kann und der gegebenenfalls an den Kohlenstoffatomen ein– bis dreifach durch $C_1$–$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino oder Ethylamino substituiert sein kann, wobei

$R^3$ für Wasserstoff, eine verzweigte oder unverzweigte Alkyl-, Alkenyl- oder Alkinylgruppe mit 1 bis 6 Kohlenstoffatomen, die gegebenenfalls durch eine oder zwei Hydroxy-, Alkoxy-, Alkylamino- oder Dialkylamino-gruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein– oder zweifach substituierter Phenacylrest, einen Oxoalkyrest mit bis zu 6 Kohlenstoffatomen steht, ferner einen Rest $COR^4$, CN oder $SO_2R^5$ bedeutet, wobei

$R^4$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen, wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino Alkylamino oder Dialkylamino mit 1 bis 5 Kohlenstoffatomen im Alkylteil und

$R^5$ geradkettiges oder verzweigtes Alkyl mit 1 bis 3 Kohlenstoffatomen darstellt, und deren pharmazeutisch verwendbaren Hydrate, Säureadditionssalze, Alkali-, Erdalkali- und Guanidiniumsalze

dadurch gekennzeichnet, dass man entweder

a) 1-Cyclopropyl-7-halogen-1,4-dihyro-4-oxo-3-chinolincarbonsäuren der Formel (II),

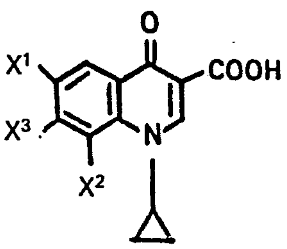

(II)

in welcher

$X^1$ und $X^2$ die oben angegebene Bedeutung haben und $X^3$ für Halogen, bevorzugt Chlor oder Fluor, steht, mit Aminen der Formel (III),

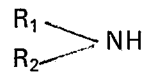

(III)

in welcher

$R^1$ und $R^2$ die oben angegebene Bedeutung haben, gegebenenfalls in Gegenwart von Säurebindern umsetzt, oder

b) eine 7-(1-Piperazinyl)-3-chinoloncarbonsäure der Formel (IV)

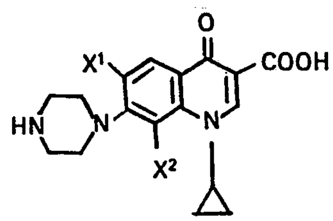

(IV)

in welcher

$X^1$ und $X^2$ die oben angegebene Bedeutung haben und der Piperazinylrest an den Kohlenstoffatomen 1–3-fach durch $C_1$–$C_4$-Alkyl, 2-Thienyl oder gegebenenfalls substituiertes Cyclohexyl oder Phenyl substituiert sein kann,

mit Verbindungen der Formel (V),

$R^3X$ (V)

in welcher

$R^3$ die oben angegebene Bedeutung hat, jedoch nicht Wasserstoff sein kann, und

X Fluor, Chlor, Brom, Jod, Hydroxy, Acyloxy, Ethoxy, Phenoxy, 4-Nitrophenoxy bedeutet, gegebenenfalls in Gegenwart von Säurebindern umsetzt, oder

c) eine 7-(1-Piperazinyl)-3-chinoloncarbonsäure der Formel (IV) in welcher der Piperazinylrest an den Kohlenstoffatomen 1 bis 3–fach durch $C_1$–$C_4$-Alkyl, 2-Thienyl oder gegebenenfalls substituiertes Cyclohexyl oder Phenyl substituiert sein kann, mit Michael-Acceptoren der Formel (VI),

$B{-}CH{=}CH_2$ (VI)

in der B für CN, CO–$R^6$ oder $COOR^7$ steht, wobei $R^6$ für Methyl oder Ethyl und $R^7$ für Methyl, Ethyl, n- oder i-Propyl steht, umsetzt.

14. Verwendung von 7-Amino-1-cyclopropyl-6,8-dihalogen- 1,4-dihydro- 4-oxo- 3-chinolincarbonsäuren gemäss den Ansprüchen 1 bis 4 bei der Herstellung von Arzneimitteln.

**Revendications**

1. Acides 7-amino-1-cyclopropyl-6,8-dihalogéno-1,4-dihydro-4-oxo-3-quinoléine-carboxyliques de formule I

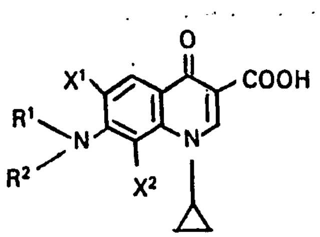

(I)

dans laquelle

$X^1$ et $X^2$, qui peuvent avoir des significations identiques ou différentes, représentent chacun le chlore ou le fluor mais ne peuvent représenter tous deux le fluor, et

$R^1$ et $R^2$ forment ensemble et avec l'atome d'azote auquel ils sont reliés un noyau hétérocyclique à 5 ou 6 chaînons qui peut contenir en outre en tant que chaînons cycliques les atomes de groupes –O–, –S–, –SO–, $-SO_2-$, N–$R^3$ ou –CO–N—$R^3$ et qui peut le cas.echéant être mono- à tri-substiué sur les atomes de carbone par des groupes alkyle en $C_1$–$C_4$, phényle eux-mêms éventuellement mono- à tri-substitués par le chlore, le fluor, le brome, des groupes méthyle, phényle, hydroxy, méthyle, benzyloxy, nitro ou pipéridino, ou cyclohexyle, 2-thiényle, hydroxy, alcoxy en $C_1$–$C_3$, amino, méthylamino ou éthylamino,

$R^3$ représente l'hydrogène, un groupe alkyle, alcényle ou alcynyle à chaîne droite ou ramifiée en $C_1$–$C_6$ qui peut le cas échéant être substitué par 1 ou 2 groupes hydroxy, alcoxy, alkylamino ou dialkylamino contenant 1 à 3 atomes de carbone dans chaque groupe alkyle, le groupe cyano, un groupe alcoxycarbonyle contenant 1 à 4 atomes de carbone dans la partie alcoolique, un groupe phénylalkyle éventuellement substitué dans la partie phényle et contenant jusqu'à 4 atomes de carbone dans la partie aliphatiques, un groupe phénacyle éventuellement mono- ou di-substitué par des groupes hydroxy, méthoxy, le chlore ou le fluor, un groupe oxoalkyle contenant jusqu'à 6 atomes de carbone, ou encore un groupe $COR^4$, CN ou $SO_2R^5$,

$R^4$ représante l'hydrogène, un groupe alkyle en $C_1$–$C_4$ à chaîne droite ou ramifiée portant éventuellement 1 ou 2 substituants choisis parmi les groupes amino, alcoxycarbonyle contenant 1 à 3 atomes de carbone dans la partie alkyle, carboxy ou alcoxy en $C_1$–$C_3$ ou les halogènes tels que le chlore, le brome, le fluor, un groupe alcoxy en $C_1$–$C_4$, amino ou alkylamino ou dialkylamino contenant 1 à 5 atomes de carbone par groupe alkyle et

$R^5$ représente un groupe alkyle à chaîne droite ou ramifiée en $C_1$–$C_3$,

et leurs hydrates, sels formés par addition avec des acides, sels alcalins, alcalino-terreux et de guanidinium acceptables pour l'usage pharmaceutique.

2. Composés de formume I de la revendication 1, dans lesquels

$X^1$ et $X^2$, qui peuvent avoir des significations identiques ou différentes, représentent le chlore ou le fluor mais ne peuvent représenter tous deux le fluor, et

$R^1$ et $R^2$ forment ensemble et avec l'atome d'azote auquel ils sont reliés un noyau hétérocyclique de 5 ou 6 chaînons qui peut contenir en outre en tant que chaînons cycliques les atomes ou groupes –O–, –S–, $-SO_2-$, N–$R^3$ ou –CO–N–$R^3$ et qui peut être substitué le cas échéant une ou deux fois sur les atomes de carbone par des groupes alkyle en $C_1$–$C_3$, cyclohexyle, phényle lui-même éventuellement mono- ou di-substitué par le chlore, le fluor, le brome, des groupes methyle, phényle, hydroxy, méthoxy, benzyloxy, nitro ou pipérdino, 2-thiényle, hydroxyamino ou méthylamino,

$R^3$ représente l'hydrogène, un groupe alkyle, alcényle ou alcynyle à chaîne droite ou ramiffiée en $C_3$–$C_4$ qui peut le cas échéant être substitué par un ou deux groupes hydroxy, un groupe phénylacyle, un groupe oxoalkyle contenant jusqu'à 5 atomes de carbone ou un groupe $COR^4$,

$R^4$ représente l'hydrogène, un groupe alkyle à chaîne droite ou ramifiée en $C_1$–$C_3$, alcoxy en $C_1$–$C_3$, amino, alkylamino ou dialkylamino contenant chacun 1 à 3 atomes de carbone par groupe alkyle.

3. Composés de formule I de la revendication 1, dans lesquels:

$X^1$ et $X^2$, qui peuvent avoir des significations identiques ou différentes, représentent le chlore ou le fluor mais ne peuvent représenter tous deux le fluor, et

$R^1$ et $R^2$ forment ensemble et avec l'atome d'azote auquel ils sont reliés un noyau hétérocyclique de 5 ou 6 chaînons qui peut contenir en outre en tant que chaînons cycliques un atome d'oxygène ou le groupe N–$R^3$ ou –CO–N–$R^3$ et qui peut le cas échéant être mono- à di-substitué sur les atomes de carbone par des groupes alkyle en $C_1$–$C_2$, cyclohexyle, phényle lui-même éventuellement substitué par le chlore, le fluor, des groupes méthyle, phenyle, hydroxy, méthoxy, benzyloxy, nitro ou pipéridino, un groupe 2-thiényle ou un groupe hydroxy,

$R^3$ représente l'hydrogène, un groupe alkyle à chaîne droite ou ramifiée en $C_1$–$C_3$ qui peut être substitué par un ou deux groupes hydroxy, un groupe phénylacyle, un groupe oxoalkyle contenant jusqu'a atomes de carbone ou un groupe $COR^4$, dans lequel

$R^4$ représente l'hydrogéne ou un groupe alkyle en $C_1$ ou $C_2$.

4. L'acide 7-(3-amino-1-pyrrolidinyl)-8-chloro-1-cyclopropyl- 6-fluoro- 1,4-dihydro-4-oxo-3-quinoléine-carboxylique.

5. L'acide 6-chloro-1-cyclopropyl-8-fluoro-1,4-dihydro-4-oxo-7- (1-pipérazinyl)- 3-quinoléine-carboxylique.

6. L'acide 6-chloro-1-cyclopropyl-7,8-difluoro-1,4-dihydro-4-oxo-3-quinoléine-carbxylique.

7. L'acide 8-chloro-1-cyclopropyl-6,7-difluoro-1,4-dihydro-4-oxo-3-quinoléine-carboxylique.

8. L'acide 6,8-dichloro-1-cyclopropyl-7-fluoro-1,4-dihydro-4-oxo-3-quinoléine-carboxylique.

9. Les acides 7-amino-1-cylopropyl-6,8-dihalogéno-1,4-dihydro-4-oxo-3-quinoléine-carboxyliques de formule I selon la revendication 1, pour l'utilisation dans le traitement de maladies.

10. Composés de formule I selon la revendication 2, pour l'utilisation dans le traitement de maladies.

11. Composés de formule I selon la revendication 3, pour l'utilisation dans le traitement de maladies.

12. Médicament caractérisé en ce qu'il contient des acides 7-amino-1-cyclopropyl-6,8-dihalogéno-1,4-dihydro-4-oxo- 3-quinoléine-carboxyliques de formule I selon la revendication 1.

13. Procédé de préparation des acides 7-amino-1cyclopropyl- 6,8-dihalogéno- 1,4-dihydro-4-oxo-3-quinoléine-carboxyliques de formule I

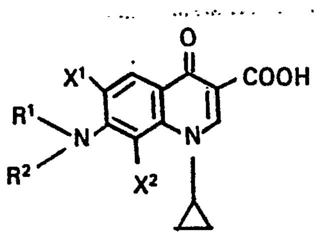

(I)

dans laquelle

$X^1$ et $X^2$, qui peuvent avoir des significations identiques ou différentes, représentent chacun le chlore ou le fluor, mais ne peuvent représenter tous deux le fluor, et

$R^1$ et $R^2$ forment ensemble et avec l'atome d'azote auquel ils sont reliés un noyau hétérocyclique de 5 ou 6 chaînons qui peut contenir en outre en tant que chaînons cycliques les atomes ou groupes –O–, –S–, –SO–, $-SO_2-$, $N-R^3$ ou –CO–$N-R^3$ et qui peut éventuellement être mono- à tri-substitué sur les atomes de carbone par des groupes alkyle en $C_1-C_4$, phényle lui-même éventuellement mono- à tri-substitué par le chlore, le fluor, le brome, des groupes méthyle, phényle, hydroxy, méthoxy, benzyloxy, nitro ou pipéridino, des groupes cyclohexyle, 2-thiényle, hydroxy-alcoxy en $C_1-C_3$, amino, méthylamino, ou éthylamino,

$R^3$ représente un atome d'hydrogène un groupe alkyle, alcényle ou alcycnyle à chaîne droite ou ramifiée en $C_1-C_6$ qui peut le cas échéant être substitué par 1 ou 2 groupes hydroxy, alcoxy, alkylamino ou dialkylamino contenant 1 à 3 atomes de carbone par groupe alkyle, le groupe cyano, un groupe alcoxycarbonyle contenant 1 à 4 atomes de carbone dans la partie alcoolique, un groupe phénylalkyle éventuellement substitué dans la partie phényle et contenant jusqu'à 4 atomes de carbone dans la partie aliphatique, un groupe phénacyle éventuellement mono- ou di-substitué par des groupes hydoxy, méthoxy, le chlore ou le fluor, un groupe oxoalkyle contenant jusqu'à 6 atomes de carbone, ou encore un groupe $COR^4$, CN ou $SO_2R^5$,

$R_4$ représente l'hydrogène, un groupe alkyle à chaîne droite ou ramifiée en $C_1-C_4$ portant éventuellement un ou deux substituants choisis parmi les groupes amino, alcoxycarbonyle contenant 1 à 3 atomes de carbone dans la partie alkyle, carboxy ou alkoxy en $C_1-C_3$ ou des halogénes tels que le chlore, le brome, le fluor, les groupes alcoxy en $C_1-C_4$, amino, alkylamino ou dialkylamino contenant 1 à 5 atomes de carbone par groupe alkyle, et

$R^5$ représente un groupe alkyle à chaîne droite ou ramifiée en $C_1-C_3$, et de leurs hydrates, sels formés par addition avec des acides, sels alcalins, alcalino-terreux et de guanidinium acceptables pour l'usage pharmaceutique, caractérise en ce que:

a) on fait réagir des acides 1-cyclopropyl-7-halogéno-1,4-dihydro- 4-oxo-3-quinoléine-carboxyliques de formule II

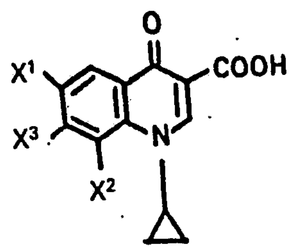

(II)

dans laquelle

$X^1$ et $X^2$ ont les significations indiquées ci-dessus et $X^3$ représente un halogéne, de préférence le chlore ou le fluor, avec des amines de formule III

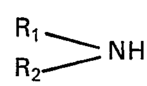

(III)

dans laquelle $R^1$ et $R^2$ ont les significations indiquées ci-dessus, éventuellement en présence de capteurs d'acides, ou bien

b) on fait réagir un acide 7-(1-pipérazinyl)-3-quinolone-carboxylique de formule IV

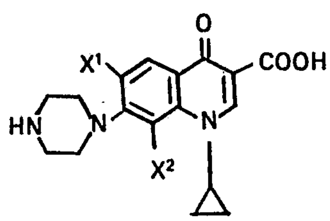

(IV)

dans laquelle

$X^1$ et $X^2$ ont les significations indiquées ci-dessus el le groupe pipérazinyle peut être substitué 1 à 3 fois sur les atomes de carbone par des groupes alkle en $C_1-C_4$, 2-thiényle ou cyclohexyle ou phényle éventuellement substitué, avec des composés de formule V

$R^3X$ (V)

dans laquelle

$R^3$ a les significations indiquées ci-dessus à

l'exception de l'hydrogène, et

X représente le fluor, le chlore, le brome, l'iode, un groupe hydroxy, acyloxy, éthoxy, phénoxy, 4-nitrophénoxy, éventuellement en présente de capteurs d'acides, ou bien

c) on fait réagir un acide 7-(1-pipéryzinyl)-3-quinolone-carboxylique de formule IV dans lequel le groupe pipérazinyl)-3-quinolone-carboxylique de formule IV dans lequel le groupe pipérazinyle peut être substitué 1 à 3 fois sur les atomes de carbone par des groupes alkyle en $C_1$–$C_4$, 2-thiényle ou cyclohexyle ou phényle éventuellement substitué, avec des accepteurs de Michael de formule VI

$$B-CH=CH_2 \qquad (VI)$$

dans laquelle B représente CN, $COR^6$ ou $COOR^7$, $R^6$ représente un groupe méthyle ou éthyle et $R^7$ représente un groupe éthyle, méthyle, n- ou isopropyle.

14. Utilisation des acides 7-amino-1-cyclopropyl-6,8-dialogéno-1,4-dihydro-4-oxo-3-quinoléine-carboxyliques selon les revendications 1 à 4 pour la préparation de médicaments.

## Claims

1. 7-Amino-1-cyclopropyl-6,8-dialogeno-1,4-dihydro-4-oxo-3-quinolinecarboxylic acids of the formula (I)

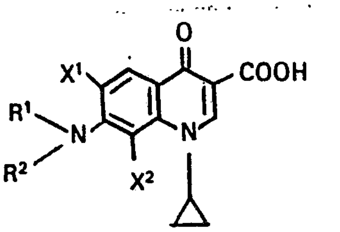

(I)

in which

$X^1$ and $X^2$, which can be identical or different, represent chlorine or fluorine, but cannot both be fluorine, and

$R^1$ and $R^2$, together with the nitrogen atom to which they are bonded, form a 5- or 6-membered heterocyclic ring which can contain in addition, as ring member, the atoms or groups –O–, –S–, –SO–, $SO_2$, $N-R^3$ or $-CO-N-R^3$ and which can optionally be monosubstituted, disubstituted or trisubstituted on the carbon atoms, by $C_1$–$C_4$-alkyl, phenyl or cyclohexyl, each of which is optionally monosubstituted, disubstituted or trisubstituted by chlorine, fluorine, bromine, methyl, phenyl, hydroxyl, methoxy, benzyloxy, nitro or piperidino, 2-thienyl, hydrooxyl, alkoxy having 1 or 3 carbon atoms, amino, methylamino or ethylamino,

$R^3$ representing hydrogen, a branched or unbranched alkyl, alkenyl or alkinyl group having 1 to 6 carbon atoms, and which can optionally be substituted by one or two hydroxyl, alkoxy, alkylamino or dialkylamino groups having 1 to 3 carbon atoms for an alkyl radical, the cyano group, or the alkoxycarbonyl group having 1 to 4 carbon atoms in the alcohol moiety, a phenylalkyl group which is optionally substituted in in the phenyl radical and has up to 4 carbon atoms in the aliphatic moiety, a phenycyl radical which is optionally monosubstituted or disubstituted by hydroxyl, methoxy, chlorine or fluorine, or an oxoalkyl radical having up to 6 carbon atoms, furthermore denoting a radical $COR^4$, CN or $SO_2R^5$,

$R^4$ representing hydrogen, straight-chain or branched alkyl which has 1 to 4 carbon atoms and is optionally substituted by 1 or 2 substituents from the group comprising amino, alkoxycarbonyl having 1 to 3 carbon atoms in the alkyl moiety, carboxyl or alkoxy having 1 to 3 carbon atoms or halogen such as chlorine, bromine or fluorine, or representing alkoxy having 1 to 4 carbon atoms, amino, alkylamino or dialkylamino having 1 to 5 carbon atoms in the alkyl moiety, and $R^5$ representing straight-chain or branched alkyl having 1 to 3 carbon atoms, and their pharmaceutically utilizable hydrates, acid addition salts, alkali metal, alkaline earth metal and guanidinium salts.

2. Compounds of the formula (I) in Claim 1, in which

$X^1$ and $X^2$, which can be indentical or different, represent chlorine or fluorine, but cannot both be fluorine, and

$R^1$ and $R^2$, together with the nitrogen atom to which they are bonded, can from a 5- or 6- membered heterocyclic ring which can contain in addition, as ring member, the atoms or groups, –O–, –S–, $-SO_2-$, $N-R^3$ or $-CO-N-R^3$, and which can optionally be monosubstituted or disubstituted on the carbon atoms by $C_1$–$C_3$-alkyl, cyclohexyl, phenyl which is optionally monosubstituted or disubstituted by chlorine, flourine, bromine, methyl, phenyl, hydroxyl, methoxy, benzyloxy, nitro or piperidino, 2-thienyl, hydroxylamino or methylamino,

$R^3$ representing hydrogen, a branched or unbranched alkyl, alkenyl or alkinyl group having 1 to 4 carbon atoms, which can optionally be substituted bs one or two hydroxyl groups, or a phenacyl radical, an oxoalkyl radical having up to 5 carbon atoms, and representing a radical $COR^4$,

$R^4$ denoting hydrogen, straight-chain or branched alkyl having 1–3 carbon atoms, alkoxy having 1–3 carbon atoms, amino, alkylamino or dialkylamino each having 1–3 carbon atoms in the alkyl moiety.

3. Compounds of the formula (I) in Claim 1, in which

$X^1$ and $X^2$, which can be indentical or different, represent chlorine or fluorine, but cannot both be fluorine, and

$R^1$ and $R^2$, together with the nitrogen atom to which they are bonded, can form a 5- or 6-membered heterocyclic ring which can contain in addition, as ring member, an oxygen atom or the groups $N-R^3$ or $-CO-N-R^3$ an which can optionally be monosubstituted or disubstituted on the carbon atoms by $C_1$–$C_2$-alkyl, cyclohexyl, phenyl

which is optionally substituted by chlorine, fluorine, methyl, phenyl, hydroxyl, methoxy, benzyloxy, nitro or piperidino, 2-thienyl or hydroxyl,

$R^3$ representing hydrogen, a branched or unbranched alkyl group having 1 to 3 carbon atoms which can optionally be substituted by one or two hydroxyl groups, or a phenacyl radical, an oxoalkyl radical having up to 4 carbon atoms and representing a radical $COR^4$,

$R^4$ denoting hydrogen or alkyl having one or two carbon atoms.

4. 7-(3-Amino-1-pyrrolidinyl)-8-chloro-1-cyclopropyl-6-fluoro-1,4-dihydro-4-oxo-3-quinolinecarboxylic acid.

5. 6-Chloro-1-cyclopropyl-8-fluoro-1,4-dihydro-4-oxo-7-(1-piperazinyl)-3-quinolinecarboxylic acid.

6. 6-Chloro-1-cyclopropyl-7,8-difluoro-1,4-dihydro-4-oxo-3-quinolinecarboxylic acid.

7. 8-Chloro-1-cyclopropyl-6,7-difluoro-1,4-dihydro-4-oxo-3-quinolinecarboxylic acid.

8. 6,8-Dichloro-1-cycloprophyl-7-fluoro-1,4-dihydro-4-oxo-3-quinolinecarboxylic acid.

9. 7-Amino-1-cyclopropyl-6,8-dialogeno-1,4-dihydro-4-oxo-3-quinolinecarboxylic acids of the formula (I) according to Claim 1 for use in combating illnesses.

10. Compounds of the formula (I) according to Claim 2 for use in combating illnesses.

11. Compounds of the formula (I) according to Claim 3 for use in combating illnesses.

12. Medicaments, characterized in that they contain 7-amino-cyclopropyl -6,8-dihalogeno-1,4-dihydro-4-oxo-3-quinolincarboxylic acids of the formula (I) according to Claim 1.

13. Process for the preparation of 7-amino-1-cyclopropyl-6,8-dihalogeno-1,4-dihydro-4-oxo-3-quinolinecarboxylic acids of the formula (I)

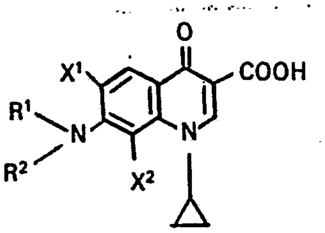

(I)

in which

$x^1$ and $X^2$, which can be identical or different, represent chlorine or fluorine, but cannot both be fluorine, and

$R^1$ and $R^2$, together with the nitrogen atom to which they are bonded, form a 5- or 6-membered heterocyclic ring which can contain in addition, as ring member, the atoms or groups –O–, –S–, –SO–, $SO_2$, N–$R^3$ or –CO–N–$R^3$ and which can optionally be monosubstituted, disubstituted or trisubstituted on the carbon atoms by $C_1$–$C_4$-alkyl, phenyl or cyclohexyl, each of which is optionally monosubstituted, disubstituted or trisubstituted by chlorine, fluorine, bromine, methyl, phenyl, hydroxyl, methoxy, benzyloxy, nitro or piperidino, 2-thienyl, hydroxyl, alkoxy having 1 to 3 carbon atoms, amino, methylamino, or ethylamino,

$R^3$ representing hydrogen, a branched or unbranched alkyl, alkenyl or alkinyl group having 1 to 6 carbon atoms, and which can optionally be substituted by one or two hydroxyl, alkoxy, alkylamino or dialkylamino groups having 1 to 3 carbon atoms for an alkyl radical, the cyano group, or the alkoxycarbonyl group having 1 to 4 carbon atoms in the alcohol moiety, a phenylalkyl group which is optionally substituted in the phenyl radical and has up to 4 carbon atoms in the aliphatic moiety, a phenacyl radial which is optionally monosubstituted or disubstituted by hydroxyl, methoxy, chlorine or fluorine, or an oxoalkyl radical having up to 6 carbon atoms, furthermore denoting a radical $COR^4$, CN or $SO_2R^5$,

$R^4$ representing hydrogen, staight-chain or branched alkyl which has 1 to 4 carbon atoms and is optionally substituted by 1 or 2 substituents from the group comprising amino, alkoxycarbonyl having 1 or 3 carbon atoms in the alkyl moiety, carboxyl or alkoxy having 1 to 3 carbon atoms, or halogen such as chlorine, bromine or fluorine, or representing alkoxy having 1 to 4 carbon atoms, amino, alkylamino or dialkylamino having 1 to 5 carbon atoms in the alkyl moiety, and

$R^5$ representing straight-chain or branched alkyl having 1 to 3 carbon atoms,

and their pharmaceutically utilizable hydrates, acid addition salts, alkali metal, alkaline earth metal and guanidinium salts, characterized in that either

(a) 1-cyclopropyl-7-halogeno-1,4-dihydro-4-oxo-3-quinoline-carboxylic acids of the formula (II)

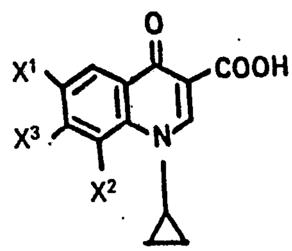

(II)

in which

$X^1$ and $X^2$ have the abovementioned meaning and

$X^3$ represents halogen, preferably chlorine or fluorine,

are reacted with amines of the formula (III)

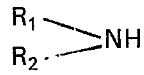

(III)

in which

$R^1$ and $R^2$ have the abovementioned meaning, where appropriate in the presence of acid-binding agents, or

(b) a 7-(1-piperazinyl)-3-quinolonecarboxylic acid of the formula (IV)

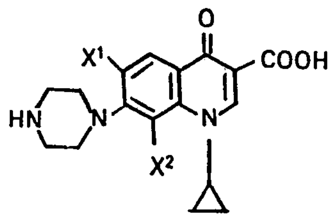

(IV)

in which

$X^1$ and $X^2$ have the abovementioned meaning, and the piperazinyl radical can be monosubstituted, disubstituted or trisubstituted on the carbon atoms by $C_1$–$C_4$-alkyl, 2-thienyl or optionally substituted cyclohexyl or phenyl, is reacted with compounds of the formula (V)

$R^3X$ (V)

in which

$R^3$ has the abovementioned meaning but cannot be hydrogen, and X denotes fluorine, chlorine, bromine, iodine, hydroxyl, acyloxy, ethoxy, phenoxy or 4-nitrophenoxy, where appropriate in the presence of acid-binding agents, or

(c) a 7-(1-piperazinyl)-3-quinolonecarboxylic acid of the formula (IV), in which the piperazinyl radical can be monosubstituted, disubstituted or trisubstituted on the carbon atoms by $C_1$–$C_4$-alkyl, 2-thienyl or optionally substituted cyclohexyl or phenyl, is reacted with Michael acceptors of the formula (VI)

$B–CH=CH_2$ (VI)

in which

B represents CN, CO–$R^6$ or $COOR^7$,

$R^6$ representing methyl or ethyl, and

$R^7$ representing methyl, ethyl, n- or i-propyl.

14. Use of 7-amino-1-cyclopropyl-6,8-dihalogeno-1,4-dihydro-4-oxo-3-quinolinecarboxylic acids according to Claims 1 to 4 in the preparation of medicaments.